# EUROPEAN PATENT SPECIFICATION

(11) **EP 4 355 306 B1**
(45) Date of publication and mention of the grant of the patent: **21.01.2026**
(21) Application number: 22734272.2
(22) Date of filing: 15.06.2022
(51) Int. Cl.: A61K 9/16, A61K 9/48, A61K 31/506

(54) **GRANULATE COMPOSITION COMPRISING NILOTINIB**
GRANULATZUSAMMENSETZUNGEN MIT NILOTINIB
COMPOSITION DE GRANULÉS COMPRENANT DU NILOTINIB

(30) Priority: 19.06.2021 EP 21180485
(43) Date of publication of application: 24.04.2024
(73) Proprietor: HELM Pharmaceuticals GmbH, 20097 Hamburg (DE)
(72) Inventor: DREYER, Katja, 22337 Hamburg (DE); COSTA, Gustavo Franco, 3046-276 Coimbra (PT); SILVA, Gabriel Leitao, 3020-229 Lordemão Coimbra (PT)
(74) Representative: Kutzenberger Wolff & Partner
(86) International application number: PCT/EP2022/066305
(87) International publication number: WO 2022/263510

(56) References cited:
- WO-A1-2012/164578
- WO-A1-2012/174082
- WO-A1-2017/158625
- CZ-A3- 2 016 785

## Description

Priority is claimed of European patent application no. 21 180 485.1 that was filed on June 19, 2021.

The invention relates to a pharmaceutical composition comprising (i) granules comprising Nilotinib, preferably in crystalline form, or a physiologically acceptable salt and/or solvate thereof, preferably crystalline anhydrous Nilotinib hydrochloride or crystalline Nilotinib hydrochloride monohydrate; and (ii) an extragranular phase comprising a surfactant, wherein the surfactant is a poloxamer and wherein essentially the total amount of surfactant that is contained in the composition is contained in the extragranular phase. The granules are dry granulated.

Nilotinib is commercialized under the tradename Tasigna^{®} in form of hard gelatin capsules. According to the summary of product characteristics (SmPC), the capsules contain Nilotinib as hydrochloride monohydrate, lactose monohydrate, crospovidone type A, poloxamer 188, colloidal anhydrous silica, and magnesium stearate. The manufacturing process of Tasigna^{®} is a standard process including aqueous wet granulation, drying, screening, mixing and encapsulation (*https:*//*www.ema.eu-ropa.eu*/*en*/*documents*/*scientific-discussion*/*tasigna-epar-scientific-discussion_en.pdf*).

EP-A 3 501 505 relates to a crystalline dispersion of nilotinib hydrochloride in a polymeric matrix, its use for the preparation of a drug form and a preparation method of this dispersion using the hot melt extrusion method.

WO 2007/015870 relates to crystalline forms of Nilotinib free base and salts thereof are prepared by various processes including Nilotinib HCl form A and Nilotinib HCl form B.

WO 2008/037716 relates to a pharmaceutical composition, especially capsules, comprising granules containing Nilotinib or a salt thereof with at least one pharmaceutically acceptable excipient. The granules may be produced by a wet granulation process.

WO 2010/054056 relates to various crystalline forms of Nilotinib hydrochloride denoted as forms T1 to T19. Nilotinib HCl crystalline form T17 is anhydrous and does not convert to other forms when heated, while other polymorphs, known in the art, such as Nilotinib HCl form A and Nilotinib HCl form B are converted to other polymorphs.

WO 2012/164578 relates to pharmaceutical compositions comprising Nilotinib or a pharmaceutically acceptable salt thereof and a process for the preparation of the same. A process for preparing dry-granulated pharmaceutical composition of Nilotinib is disclosed comprising compacting nilotinib hydrochloride alone or mixing with one or more of pharmaceutically acceptable excipient(s) by roller compactor or slugging; sizing the compacts or slugs into granules by milling; and mixing the granules with one or more of pharmaceutically acceptable excipients to form the composition. In some of the examples of WO 2012/164578, poloxamer is used as an intragranular surfactant.

WO 2012/174082 relates to soluble pharmaceutical compositions of amorphous nilotinib or a pharmaceutically acceptable salt thereof using one or more organic acids that function as a solubilizing agent, increasing the bioavailability of nilotinib and suppressing the food effect associated with certain compositions of nilotinib. A spray dried mixture of Nilotinib and several excipients is subsequently mixed with poloxamer and then roller compacted such that poloxamer is present as an intragranular surfactant.

WO 2017/064538 relates to a composition comprising nilotinib hydrochloride anhydrous form T17, wherein crospovidone is used as filler in the formulation instead of prior art fillers like microcrystalline cellulose and lactose. In the examples of WO 2017/064538, poloxamer is used as an intragranular surfactant. Further, WO 2017/064538 reports that during formulation studies it was found that Nilotinib HCl crystalline form T17 is not compatible with most excipients used in pharmaceutical formulations. Especially, common diluents and fillers such as lactose anhydrous, lactose monohydrate, cellulosic excipients such as microcrystalline cellulose, mannitol used in prior art formulations (WO 2008/037716, WO 2012/164578, WO 2014/060449, WO 2014/174496) comprising Nilotinib have been observed to be incompatible with nilotinib anhydrous form T17. Hence the resulting formulation experiences high polymorphic conversion.

WO 2017/158625 relates to a method of treating leukaemia comprising orally administering to a patient in need thereof reduced daily doses of nilotinib of 100 mg to 600 mg, wherein the nilotinib is administered in a dosage form having a composition comprising nilotinib butanedisulphonate (2:1) or nilotinib butanedisulphonate (1:1).

WO 2020/095187 relates to a crystalline form of nilotinib hydrochloride, process for its preparation and pharmaceutical composition comprising the same.

CZ 2 016 785 relates to a pharmaceutical composition comprising nilotinib in the form of minitablets.

N.J. Koehl et al., Mol. Pharmaceutics 2020, 17, 9, 3202-3213, reports about an investigation exploring the impact of surfactant type and digestion and reveals that highly digestible surfactants improve oral bioavailability of Nilotinib. Medium-chain fatty acid-based surfactants are found to be more readily digested compared to long-chain surfactants, and for Nilotinib highly digestible, medium chain-based surfactants are preferred.

The known pharmaceutical compositions and dosage forms of Nilotinib are not satisfactory in every respect and there is a demand for alternative or improved pharmaceutical compositions and dosage forms of Nilotinib, especially of crystalline anhydrous Nilotinib hydrochloride or crystalline Nilotinib hydrochloride monohydrate.

It is an object of the invention to provide pharmaceutical compositions and dosage forms of Nilotinib that have advantages over the prior art.

This object has been achieved by the subject-matter of the patent claims.

It has been surprisingly found that pharmaceutical compositions and dosage forms can be prepared that are bioequivalent to commercial Tasigna^{®} and that contain Nilotinib and surfactant in separate phases, namely Nilotinib in the intragranular phase and the surfactant in the extragranular phase.

Formulating poorly water-soluble drugs such as Nilotinib and surfactants such as poloxamer in separate phases (intragranular vs. extragranular) is unusual. One would typically expect that the surfactant can evolve its solubilizing effect only when it is intimately mixed with the drug (i.e. intragranularly), but not when it is formulated in a separate phase (i.e. extragranularly). The possible impact of the surfactant at particle surfaces in the microenvironment around the dissolving particles where local concentrations are high should be considered.

This is also the reason why e.g. in hydrophilization processes a solution of a hydrophilizing excipient is spread onto a drug in a high-shear mixer in order to achieve an intimate mixture of drug and hydrophilizing excipient; the resultant mixture is then dried and screened. Usually no benefit is accrued, however, when drug and hydrophilizing excipient are merely dry blended (see e.g. A.T. Florence et al., Modern Pharmaceutics, Volume 1, CRC Press 2010, page 543).

Various methods for the preparation of poloxamer-based drug delivery formulations are known which all have in common that the drug is intimately mixed with the poloxamer, such as the methods referred to as (i) direct solubilization of actives, (ii) thin film hydration, (iii) temperature-induced emulsification, (iv) solvent displacement, or (v) kinetically frozen micelles (A.M. Bodratti et al., J. Funct. Biomater. 2018, 9, 11, 1-24).

It is common general knowledge that surfactants such as poloxamers enhance the bioavailability of the drug, and that the granulation process can ensure an intimate mixture of drug and surfactant merely by the mixing energy of the process, or by putting both the drug and surfactant in the liquid used for granulating (see e.g. D.M. Parkih, Handbook of Pharmaceutical Granulation Technology, 4th ed., CRC Press 2021, page 136).

Surfactants are also used in solid drug dispersions as additives that alter dissolution via a mechanism other than the thermodynamic solubility advantage produced by an amorphous dispersion of a poorly soluble drug in a suitable matrix. A common approach is inclusion of a surfactant component such as poloxamer within the dispersion (i.e., intragranular) to enhance the wetting, disintegration, and dissolution of solid dispersed drug into solution (see e.g. A. Newman, Pharmaceutical Amorphous Solid Dispersions, Wiley 2015, page 345). Studies show that surfactants like poloxamer added extragranularly to solid dispersions typically have minimal effect on the drug release (J.P. Lakshman et al., Mol. Pharm. 5 (2008) 994-1002). However, when the surfactants are intimately mixed with solid dispersion, i.e. when surfactant, matrix polymer and drug are dissolved in common solvent and dried to yield solid dispersion, they have shown a positive effect on drug release. When surfactants are incorporated in the solid dispersion, the drug remains in the vicinity of the surfactant during dissolution creating micro-environment rich with surfactant increasing the drug solubility (S.P. Chaudhari et al., Journal of Drug Delivery Science and Technology 41 (2017) 68-77).

Therefore, formulating the surfactant e.g. poloxamer in the extragranular phase would typically not be considered as a straightforward approach for formulating Nilotinib.

Further, it has been surprisingly found that the surfactant in the extragranular phase may be homogeneously distributed by blending the granules with the extragranular phase, especially by geometric dilution. Without wishing to be bound to any scientific theory, it seems that surfactant may be blended with dry granulated granules containing the Nilotinib in a manner where surfactant deposits on the surface of the granules and develop their solubilizing activity when the granules come into contact with gastric juice. Thus, although the surfactant is not intimately mixed with the Nilotinib, the surfactant can nevertheless evolve its beneficial effect with regard to solubilizing the poorly soluble drug of Class IV and to ensure adequate in-vivo behavior. This can be achieved with both, non-micronized or micronized surfactant.

Still further, it has been surprisingly found that pharmaceutical compositions and dosage forms can be prepared that are stable and that contain in the intragranular phase anhydrous lactose in a mixture with Nilotinib, preferably anhydrous Nilotinib hydrochloride form T17, which according to WO 2017/064538 is said to be incompatible with anhydrous lactose. The present invention makes it possible to use anhydrous lactose and to keep it anhydrous throughout the shelf-life of the pharmaceutical composition.

Furthermore, it has been surprisingly found that pharmaceutical compositions can be prepared by an anhydrous granulation process (i.e. dry granulation) using surfactant such as poloxamer in the extragranular phase and nonetheless ensuring satisfactory and decent *in vivo* data with acceptable inter subject variability.

Moreover, it has been found that the employed form of Nilotinib, preferably anhydrous Nilotinib hydrochloride form T17 or Nilotinib hydrochloride monohydrate form B, can be maintained during an anhydrous granulation process (i.e. dry granulation).

A first aspect of the invention relates to a pharmaceutical composition comprising
(i) granules comprising Nilotinib, preferably in crystalline form, or a physiologically acceptable salt and/or solvate thereof, preferably crystalline anhydrous Nilotinib hydrochloride or crystalline Nilotinib hydrochloride monohydrate; and
(ii) an extragranular phase;

wherein the granules are dry-granulated;
wherein the pharmaceutical composition comprises a surfactant which is contained in the extragranular phase; wherein the surfactant is a poloxamer, preferably non-micronized or micronized poloxamer.

Unless expressly stated otherwise, all percentages are weight percent (wt.-%).

Unless expressly stated otherwise, all editions and supplements of pharmacopoeia refer to the version that is valid on January 1, 2021.

Unless expressly stated otherwise, *"essentially consisting of"* means at least 95 wt.-%, preferably at least 98 wt.-%, more preferably at least 99 wt.-%.

As used herein, the term *"anhydrous"* refers to a crystalline form having less than about 2 wt.- % total water (bound and unbound).

As used herein, the term *"monohydrate"* refers to a crystalline form having at least one molecule of water per molecule of Nilotinib (bound and unbound).

The pharmaceutical composition according to the invention comprises an intragranular phase and an extragranular phase, wherein the granules comprising Nilotinib form the intragranular phase.

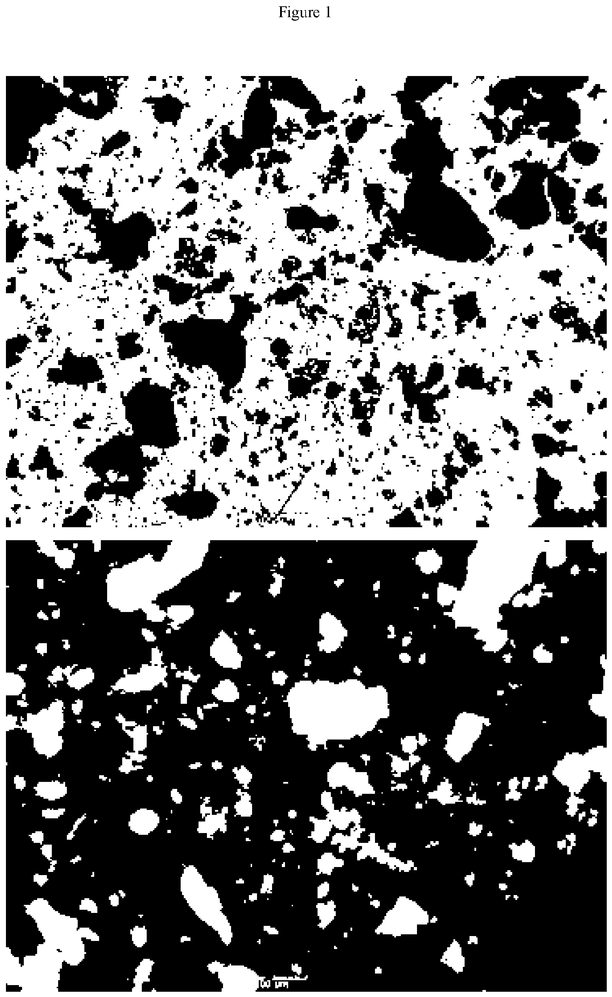

Figures 1 to 4 show photographs (10X magnitude) of various pharmaceutical formulations which were prepared by blending different types of poloxamer (coarse or micronized) with granules under different blending conditions (direct addition vs. geometric dilution). **In** each case the upper photograph was taken with polarized light, the lower photograph with non-polarized light.
Figure 1 shows a comparative pharmaceutical formulation where the granules were blended with non-micronized poloxamer by direct addition.
Figure 2 shows another comparative pharmaceutical formulation where the granules were blended with micronized poloxamer by direct addition.
Figure 3 shows an inventive pharmaceutical formulation where the granules were blended with non-micronized poloxamer by geometric dilution.
Figure 4 shows another inventive pharmaceutical formulation where the granules were blended with micronized poloxamer by geometric dilution.

The pharmaceutical composition according to the invention comprises a surfactant that is contained in the extragranular phase. Essentially the total amount of surfactant that is contained in the composition is contained in the extragranular phase.

In preferred embodiments, the surfactant is solid but not micronized, i.e. a coarse particulate material. According to the invention, the non-micronized surfactant is homogeneously distributed over the extragranular phase of the pharmaceutical composition. Preferably, in the course of manufacture of the pharmaceutical composition, the non-micronized surfactant is added by geometric dilution thereby achieving the desired degree of homogeneity of its distribution.

In other preferred embodiments, the surfactant is a micronized surfactant. Micronized surfactants are known to the skilled person and commercially available, e.g. micronized poloxamers. For the purpose of the specification, *"micronized"* refers to a powder having a weight average particle size on the micrometer scale. According to the invention, the micronized surfactant is also homogeneously distributed over the extragranular phase of the pharmaceutical composition. Preferably, in the course of manufacture of the pharmaceutical composition, the micronized surfactant is added by geometric dilution thereby achieving the desired degree of homogeneity of its distribution.

Preferably, the surfactant is homogeneously distributed such that the relative standard deviation (RSD) of the surfactant content in a multitude of samples of the pharmaceutical composition according to the invention, preferably in a multitude of 5 samples, more preferably in a multitude of 10 samples, is not more than 10.9%, preferably more than 10.6%, more preferably more than 10.3%, still more preferably more than 10.0%, yet more preferably more than 9.7%, even more preferably more than 9.4%, most preferably more than 9.1%, and in particular more than 8.8%. Preferably, when performing the test, one sample has a weight of about 1.0 g. Preferably, the poloxamer content is quantified by HPLC, more preferably by HPLC-RI, in particular in accordance with Y. Mao et al., Quantitation of poloxamers in pharmaceutical formulations using size exclusion chromatography and colorimetric method, Journal of Pharmaceutical and Biomedical Analysis, 35 (2004) 1127-1142.

In preferred embodiments, the micronized surfactant has a weight average particle size as measured by sieve analysis in accordance with Ph. Eur. 2.9.38, of 50±40 µm, preferably 50±35 µm, more preferably 50±30 µm, still more preferably 50±25 µm, yet more preferably 50±20 µm, even more preferably 50±15 µm, most preferably 50±10 µm, and in particular 50±5 µm.

In preferred embodiments, the micronized surfactant has a particle size distribution as measured by sieve analysis in accordance with Ph. Eur. 2.9.38, wherein not more than 10 wt.-% retain on a sieve having a size of≤106 µm, and not more than 50 wt.-% retain on a sieve having a size of≤53 µm.

The surfactant is selected from the group consisting of poloxamers.

Preferably, the surfactant is a solid at room temperature. For the purpose of the specification, surfactants that are solid at room temperature (i.e. 23 °C) include surfactants that are waxy materials.

Preferably, the surfactant has a HLB value within the range of 29±8, preferably 29±7, more preferably 29±6, still more preferably 29±5, yet more preferably 29±4, even more preferably 29±3, most preferably 29±2, and in particular 29±1.

The surfactant is a poloxamer; preferably poloxamer 188 or poloxamer 407. Poloxamer 188 and 407 are commercially available, e.g. under the trade name Kolliphor^{®}, Lutrol^{®} F68, Pluronic^{®} F-68, and Synperonic^{®} F68, and the like.

Poloxamer 188 is known to the skilled person and commercially available. Poloxamer 188 satisfies the following formula HO-[-CH₂CH₂-O-]ₐ-[-CH₂CH(CH₃)-O-]_{b}-[-CH₂CH₂-O-]ₐ-H, wherein index a is approximately 79 and index b is approximately 28, whereas the proportion of polyoxyethylene is approximately 70 wt.-%. In poloxamer 407, index a is approximately 101 and index b is approximately 56, whereas the proportion of polyoxyethylene is approximately 80 wt.-%.

Essentially the total amount of surfactant that is contained in the composition according to the invention is contained in the extragranular phase, i.e. the granules contain no surfactant.

Preferably, the content of the surfactant is within the range of from 0.5 to 1.5 wt.-%, preferably 0.70±0.20 wt.-%, more preferably 0.80±0.20 wt.-%, still more preferably 0.90±0.20 wt.-%, yet more preferably 1.00±0.20 wt.-%, relative to the total weight of the composition.

The pharmaceutical composition according to the invention comprises Nilotinib (C₂₈H₂₂F₃N₇O, ATC L01EA03), i.e. 4-methyl-N-[3-(4-methyl-1H-imidazol-1-yl)-5-(trifluoromethyl)phenyl]-3-{[4-(pyridin-3-yl)pyrimidin-2-yl]amino}benzamide:

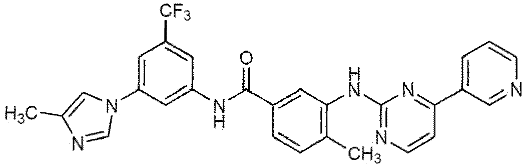

Unless expressly stated otherwise, all percentages and dosages are expressed as equivalents based upon the weight of Nilotinib in its non-salt and non-solvate form, i.e. C₂₈H₂₂F₃N₇O.

Preferably, the Nilotinib is crystalline.

Preferably, the pharmaceutical composition according to the invention comprises no amorphous Nilotinib, physiologically acceptable salt and/or solvate thereof.

Preferably, the Nilotinib is anhydrous or a hydrate.

Preferably, the Nilotinib is anhydrous; preferably a Nilotinib ansolvate.

Preferably, the Nilotinib is a hydrate; preferably a monohydrate.

Preferably, the Nilotinib is a Nilotinib hydrochloride salt.

In particularly preferred embodiments of the invention, the Nilotinib is crystalline anhydrous Nilotinib hydrochloride salt; preferably Nilotinib HCl form T17. Nilotinib HCl form T17 is described e.g. in WO 2010/054056.

Nilotinib HCl form T17 is characterized by data selected from the group consisting of: (i) an x-ray powder diffraction pattern having peaks at 5.7, 9.8, 15.0, 15.8 and 17.3 degrees two theta ± 0.2 degrees two theta; preferably having additional peaks at 7.5, 11.4, 18.6, 19.6 and 20.7 degrees two theta ± 0.2 degrees two theta, or at 7.6, 11.4, 18.7, 19.7 and 20.7 degrees two theta ± 0.2 degrees two theta; (ii) a solid-state ¹³C NMR spectrum with signals at 113.1, 133.1, 160.9 ± 0.2 ppm; (iii) a solid-state ¹³C NMR spectrum having chemical shifts differences between the signal exhibiting the lowest chemical shift and another in the chemical shift range of 100 to 180 ppm of 9.2, 29.2 and 57.0 6 0.1 ppm, wherein the signal exhibiting the lowest chemical shift in the chemical shift area of 100 to 180 ppm is typically at about 103.9 ± 0.2 ppm; and combinations thereof.

Anhydrous Nilotinib hydrochloride is characterized as a Class IV compound (low / moderate aqueous solubility and low permeability) according to the Biopharmaceutical Classification System (BCS).

In other preferred embodiments of the invention, the Nilotinib is crystalline Nilotinib hydrochloride monohydrate salt; preferably Nilotinib HCl form B. Nilotinib HCl form B is described e.g. in WO 2007/015870.

Nilotinib HCl form B is characterized by an x-ray powder diffraction pattern having at least one maxima selected from 7.2, 9.2, 11.4, 12.0, 12.3, 14.6, 14.8, 15.7, 17.6, 19.2, 19.5, 20.5, 22.0, 23.4, 23.9, 25.0, 25.5, 25.9 and 27.0 degrees two theta.

Preferably, the content of Nilotinib is within the range of 50±35 wt.-%, preferably 50±30 wt.-%, more preferably 50±25 wt.-%, still more preferably 50±20 wt.-%, yet more preferably 50±15 wt.-%, even more preferably 50±10 wt.-%, most preferably 50±5.0 wt.-%, and in particular 50±2.5 wt.-%, relative to the total weight of the composition and expressed as equivalent weight of Nilotinib in its non-salt non-solvate form.

The granules and/or the extragranular phase of the pharmaceutical composition according to the invention may comprise one or more pharmaceutical excipients. Preferably, the pharmaceutical excipients are selected from diluents (fillers), disintegrants, glidants, lubricants, surfactants, binders, antioxidants, preservatives, colorants, pigments, dyes, pH modifiers, and the like.

Preferably, the granules of the pharmaceutical composition according to the invention are composed of a granulated mixture of all ingredients, i.e. of Nilotinib and all intragranular excipients, in neat form, but is not composed of a mixture of a preformulated blend with intragranular excipients. For example, the granules of the pharmaceutical composition according to the invention are preferably not composed of a spray dried formulation comprising Nilotinib and one or more excipients (preformulated blend) in admixture with further excipients.

Preferably, the granules of the composition according to the invention comprise a diluent.

Preferably, the diluent is selected from the group consisting of lactose, glucose, sucrose, sorbitol, mannitol, dextrates, dextrin, dextrose, microcrystalline cellulose, and powdered cellulose; preferably lactose.

In particularly preferred embodiments, the diluent is anhydrous; preferably anhydrous lactose.

Preferably, the content of the diluent is within the range of 40±35 wt.-%, preferably 40±30 wt.- %, more preferably 40±25 wt.-%, still more preferably 40±20 wt.-%, yet more preferably 40±15 wt.-%, even more preferably 40±10 wt.-%, most preferably 40±5.0 wt.-%, and in particular 40±2.5 wt.-%, relative to the total weight of the composition.

Preferably, essentially the total amount of diluent that is contained in the composition according to the invention is contained in the granules, i.e. the extragranular phase preferably contains no diluent.

Preferably, the granules of the composition according to the invention comprise a disintegrant.

Preferably, the disintegrant is selected from the group consisting of cross-linked polyvinyl pyrrolidone, cross-linked sodium carboxymethylcellulose, cross-linked calcium carboxymethylcellulose, pregelatinized starch, clay, cellulose, soy polysaccharides, alginates, and gums.

In particularly preferred embodiments, the disintegrant is cross-linked polyvinyl pyrrolidone; preferably crospovidone according to Ph. Eur. crospovidone monograph type A. Examples and commercial grades of crospovidone can be used, e.g. Kollidon^{®} CL, Kollidon^{®} CL-M, Polyplasdone^{®} XL, Polyplasdone^{®} XL-10, Polyplasdone^{®} Ultra. Preferably crospovidone used in the composition has a mean particle size of less than 150 µm.

Preferably, the disintegrant has a weight average particle size as measured by sieve analysis in accordance with Ph. Eur. 2.9.38, of 125±50 µm, preferably 125±45 µm, more preferably 125±40 µm, still more preferably 125±35 µm, yet more preferably 125±30 µm, even more preferably 125±25 µm, most preferably 125±20 µm, and in particular 125±15 µm.

Preferably, the content of the disintegrant is within the range of 4.0±3.5 wt.-%, preferably 4.0±3.0 wt.-%, more preferably 4.0±2.5 wt.-%, still more preferably 4.0±2.0 wt.-%, yet more preferably 4.0±1.6 wt.-%, even more preferably 4.0±1.4 wt.-%, most preferably 4.0±1.2 wt.-%, and in particular 4.0±1.0 wt.-%, relative to the total weight of the composition.

Preferably, essentially the total amount of disintegrant that is contained in the composition according to the invention is contained in the granules, i.e. the extragranular phase preferably contains no disintegrant.

Preferably, the pharmaceutical composition according to the invention comprises a glidant.

In preferred embodiments, the granules comprise a glidant. In other preferred embodiments, the extragranular phase comprises a glidant. In further preferred embodiments, the granules as well as the extragranular phase comprise a glidant, which is preferably the same glidant.

Preferably, the glidant is selected from the group consisting of colloidal silica, magnesium trisilicate, starch, talc, tribasic calcium phosphate, magnesium carbonate, magnesium oxide and powdered cellulose; preferably colloidal silica.

In particularly preferred embodiments, the glidant is anhydrous colloidal silica.

Preferably, the content of the glidant is within the range of 1.0±0.9 wt.-%, preferably 1.0±0.8 wt.-%, more preferably 1.0±0.7 wt.-%, still more preferably 1.0±0.6 wt.-%, yet more preferably 1.0±0.5 wt.-%, even more preferably 1.0±0.4 wt.-%, most preferably 1.0±0.3 wt.-%, and in particular 1.0±0.2 wt.-%, relative to the total weight of the composition.

In preferred embodiments, a first fraction of the glidant is contained in the granules and a second fraction (the remainder) of the glidant is contained in the extragranular phase.

Preferably, the pharmaceutical composition according to the invention comprises a lubricant.

In preferred embodiments, the granules comprise a lubricant. In other preferred embodiments, the extragranular phase comprises a lubricant. In further preferred embodiments, the granules as well as the extragranular phase comprise a lubricant, which is preferably the same lubricant.

Preferably, the lubricant is selected from the group consisting of magnesium stearate, aluminum stearate, calcium stearate, polyethylene glycol, glyceryl behenate, stearic acid, hydrogenated castor oil, glyceryl monostearate and sodium stearyl fumarate.

In particularly preferred embodiments, the lubricant is magnesium stearate.

Preferably, the content of the lubricant is within the range of 1.5±1.3 wt.-%, preferably 1.5±1.1 wt.-%, more preferably 1.5±0.9 wt.-%, still more preferably 1.5±0.7 wt.-%, yet more preferably 1.5±0.5 wt.-%, even more preferably 1.5±0.3 wt.-%, most preferably 1.5±0.2 wt.-%, and in particular 1.5±0.1 wt.-%, relative to the total weight of the composition.

In preferred embodiments, a first fraction of the lubricant is contained in the granules and a second fraction (the remainder) of the lubricant is contained in the extragranular phase.

In preferred embodiments, the pharmaceutical composition according to the invention comprises no povidone; preferably no binder selected from the group consisting of povidone, microcrystalline cellulose, hydroxyethylcellulose, hydroxypropylcellulose, and hydroxypropylmethylcellulose.

In preferred embodiments, the granules essentially consist of Nilotinib or a physiologically acceptable salt and/or solvate thereof.

In other preferred embodiments, the granules essentially consist of Nilotinib, namely in crystalline form, or a physiologically acceptable salt and/or solvate thereof, namely crystalline anhydrous Nilotinib hydrochloride or crystalline Nilotinib hydrochloride monohydrate, diluent, disintegrant, optionally glidant, and optionally lubricant.

In preferred embodiments, the extragranular phase contains no lactose; preferably no diluent selected from the group consisting of lactose, glucose, sucrose, sorbitol, mannitol, dextrates, dextrin, dextrose, microcrystalline cellulose, and powdered cellulose.

In preferred embodiments, the extragranular phase essentially consists of surfactant, glidant and lubricant.

The granules are dry granulated.

In particularly preferred embodiments of the pharmaceutical composition according to the invention
(i) the granules comprise or essentially consist of
   - crystalline anhydrous Nilotinib hydrochloride or crystalline Nilotinib hydrochloride monohydrate;
   - diluent, preferably anhydrous lactose;
   - disintegrant, preferably cross-linked polyvinylpyrrolidone;
   - a fraction of glidant; preferably anhydrous colloidal silica; and
   - a fraction of lubricant; preferably magnesium stearate; and
(ii) the extragranular phase comprises or essentially consists of
   - poloxamer; preferably poloxamer 188 and/or micronized poloxamer;
   - the remainder of glidant; preferably anhydrous colloidal silica;
   - the remainder of lubricant; preferably magnesium stearate.

Preferably, the pharmaceutical composition according to the invention does not contain citric acid or a salt thereof.

Preferably, the pharmaceutical composition according to the invention does not contain an organic acid selected from acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, glutamic acid, aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid and ascorbic acid or a salt thereof; preferably no organic acid at all or any salt thereof.

Preferably, the granules that are contained in the pharmaceutical composition according to the invention have a size such that they pass a sieve having a mesh size of 1.0 to 2.0 mm but do not pass a sieve having a mesh size of 0.5 mm; preferably 0.6 mm, more preferably 0.7 mm, still more preferably 0.8 mm.

Preferably, the granules have an essentially spherical shape, although other shapes are contemplated as well.

Another aspect of the invention relates to a process for the preparation of a pharmaceutical composition according to the invention as described above comprising the step of dry granulating a formulation comprising the Nilotinib, preferably in crystalline form, or a physiologically acceptable salt and/or solvate thereof, preferably crystalline anhydrous Nilotinib hydrochloride or crystalline Nilotinib hydrochloride monohydrate.

Dry granulating according to the invention involves either slugging or roller compaction.

Slugging is a compaction process in which the material to be processed is compressed to a large compressed mass, or *"slug,"* which is further milled to form granules and are finally filled into capsules or compressed into tablets. The slugging process typically includes (i) sifting and blending of dry mix ingredients; (ii) compressing the blend of step (i) to obtain slugs; (iii) milling and sifting the step of (ii) to obtain granules; (iv) lubricating and blending the granules of step (iii), and finally filling into capsules or compressing into tablets.

Roller compaction is a process by which two or more solid materials are compacted between rotating rolls, desirably counter-rotating rolls, to form solid ribbons/compacts. The resulted compacts are typically sized into granules by milling to modify the desired particle size; and optionally the granules are mixed with one or more of pharmaceutically acceptable excipients to form the composition.

The process according to the invention preferably comprises compacting Nilotinib, preferably in crystalline form, or a physiologically acceptable salt and/or solvate thereof, preferably crystalline anhydrous Nilotinib hydrochloride or crystalline Nilotinib hydrochloride monohydrate, alone or mixing with one or more of pharmaceutically acceptable excipient(s) by roller compactor or slugging; sizing the compacts or slugs into granules by milling; and mixing the granules with one or more of pharmaceutically acceptable excipients to form the final composition. The mixing process preferably includes: (i) sifting and blending of Nilotinib, preferably in crystalline form, or a physiologically acceptable salt and/or solvate thereof, preferably crystalline anhydrous Nilotinib hydrochloride or crystalline Nilotinib hydrochloride monohydrate, with one or more pharmaceutically acceptable excipients to form a dry mixture (pharmaceutical composition); (ii) followed by filling the dry mixture of step (i) into capsules (pharmaceutical dosage form).

In preferred embodiments, the process comprises the steps of
(a) providing a formulation comprising Nilotinib, preferably in crystalline form, or a physiologically acceptable salt and/or solvate thereof, preferably crystalline anhydrous Nilotinib hydrochloride or crystalline Nilotinib hydrochloride monohydrate, optionally diluent, optionally disintegrant, optionally a fraction of glidant, and optionally a fraction of lubricant;
(b) dry granulating the formulation thereby obtaining granules;
(c) optionally, sieving the granules;
(d) providing a surfactant formulation comprising or essentially consisting of surfactant, wherein the surfactant is poloxamer; and
(e) blending the surfactant formulation and the granules.

In step (a) of the process according to the invention, a formulation is provided that comprises Nilotinib, preferably in crystalline form, or a physiologically acceptable salt and/or solvate thereof, preferably crystalline anhydrous Nilotinib hydrochloride or crystalline Nilotinib hydrochloride monohydrate, and optionally one or more of the following, preferably all of the following: diluent, disintegrant, a fraction of glidant, and a fraction of lubricant.

All ingredients of the formulation are preferably weighed. Preferably, in a first sub-step, all excipients are mixed and sifted, and subsequently the Nilotinib, preferably in crystalline form, or a physiologically acceptable salt and/or solvate thereof, preferably crystalline anhydrous Nilotinib hydrochloride or crystalline Nilotinib hydrochloride monohydrate is added. The mixture is then preferably blended, e.g. at a rotational speed of the blender within the range of from 3 to 9 rpm, more preferably 5 to 7 rpm. The blending time is not particularly limited. Blending typically lasts 5 to 15 minutes, preferably 8 to 12 minutes.

In step (b) of the process according to the invention, the formulation provided in step (a) is dry granulated thereby obtaining granules.

Preferably, dry granulating is performed by roller compaction.

Preferably, roller compaction is performed at an average roller gap within the range of from 0.5 to 2.0 mm; preferably 1.0±0.2 mm.

Preferably, roller compaction is performed at a pressure within the range of from 20 to 40 bar; preferably 25 to 35 bar.

In optional step (c) of the process according to the invention, the granules are sieved thereby obtaining calibrated granules.

Preferably, sieving the granules in step (c) calibrates large granules not passing a sieve of size 1.60 mm as well as small granules passing a sieve of size 0.80 mm.

In step (d) of the process according to the invention, a surfactant formulation is provided that comprises or essentially consisting of surfactant, wherein the surfactant is poloxamer. Preferably, the surfactant formulation essentially consists of poloxamer.

In optional step (e) of the process according to the invention, the granules obtained in step (b), or preferably the calibrated granules obtained in optional step (c), are blended with the surfactant formulation provided in step (d).

Preferably, in optional step (e) in an initial sub-step the surfactant formulation is blended with a fraction of the granules, and wherein in subsequent sub-steps additional fractions of the granules are added to the blend (geometric dilution process).

Thus, the granules obtained in step (b), or preferably the calibrated granules obtained in optional step (c), are preferably divided into fractions, preferably having approximately the same weight. Preferably, the granules are divided into 2, 3, 4, 5, 6, 7, 8, 9, or 10 fractions. Preferably, in an initial sub-step the surfactant formulation is blended with a first fraction of the granules, in a subsequent sub-step a second fraction of the granules is added to the blend and the thus obtained mixture is further blended, in another subsequent sub-step a third fraction of the granules is added to the blend and the thus obtained mixture is further blended, and so on (geometric dilution process).

Preferably, optional step (e) comprises the sub-steps
(e₁) blending the surfactant formulation with a first fraction of the granules thereby obtaining a first blend; and optionally sieving the first blend;
(e₂) blending the first blend with a second fraction of the granules thereby obtaining a second blend; optionally sieving the second blend;
(e₃) optionally, blending the second blend with a third fraction of the granules thereby obtaining a third blend; optionally sieving the third blend;
(e₄) optionally, blending the third blend with a fourth fraction of granules thereby obtaining a fourth blend; optionally sieving the fourth blend; and
(e₅) optionally, blending the fourth blend with a fifth fraction of granules thereby obtaining a fifth blend; optionally sieving the fifth blend.

Preferably, every blending step, i.e. (e₁), (e₂), and so on is performed for 0.5 to 2 minutes, preferably 1 minute.

Preferably, in optional step (e) the surfactant is blended with the granules until a homogeneous distribution is achieved such that the relative standard deviation (RSD) of the surfactant content in a multitude of samples, preferably in a multitude of 5 samples, more preferably in a multitude of 10 samples, is not more than 10.9%, preferably more than 10.6%, more preferably more than 10.3%, still more preferably more than 10.0%, yet more preferably more than 9.7%, even more preferably more than 9.4%, most preferably more than 9.1%, and in particular more than 8.8%. Preferably, when performing the test, one sample has a weight of about 1.0 g. Preferably, the poloxamer content is quantified by HPLC, more preferably by HPLC-RI.

Sieving the blends is preferably performed by means of a sieve having a size of 0.5 to 1.0 mm, e.g. 0.7 mm.

In preferred embodiments, the process comprises the additional steps of blending the granules obtained in step (b), the sieved granules obtained in step (c) or the product obtained in step (e)
(f) with the remainder of glidant; and optionally, sieving the thus obtained blend; and/or
(g) with the remainder of lubricant; and optionally, sieving the thus obtained blend.

In step (f) of the process according to the invention, the granules obtained in step (b), the sieved granules obtained in optional step (c) or the product obtained in optional step (e) is blended with glidant. Preferably, the glidant is the same glidant that is also contained in the granules, i.e. preferably the granules obtained in step (b), the sieved granules obtained in optional step (c), or the product obtained in optional step (e) is blended with the remainder of glidant.

The mixture is then preferably blended, e.g. at a rotational speed of the blender within the range of from 3 to 9 rpm, more preferably 5 to 7 rpm. The blending time is not particularly limited. Blending typically lasts 1 to 10 minutes, preferably 3 to 7 minutes.

In step (g) of the process according to the invention, the granules obtained in step (b), the sieved granules obtained in optional step (c), the product obtained in optional step (e) or the product obtained in step (f) is blended with lubricant. Preferably, the lubricant is the same lubricant that is also contained in the granules, i.e. preferably the granules obtained in step (b), the sieved granules obtained in optional step (c), the product obtained in optional step (e) or the product obtained in step (f) is blended with the remainder of lubricant.

The mixture is then preferably blended, e.g. at a rotational speed of the blender within the range of from 3 to 9 rpm, more preferably 5 to 7 rpm. The blending time is not particularly limited. Blending typically lasts 1 to 5 minutes, preferably 2 to 4 minutes.

Preferably, in the process according to the invention, the form of Nilotinib provided in step (a) is maintained in the product.

Another aspect of the invention relates to a pharmaceutical composition that is obtainable by the process according to the invention as described above.

Another aspect of the invention relates to a pharmaceutical dosage form comprising the pharmaceutical composition according to the invention as described above.

Preferably, the pharmaceutical dosage form according to the invention is devoted for oral administration.

Preferably, the pharmaceutical dosage form according to the invention is a capsule; preferably a hard gelatin capsule. Preferably, the pharmaceutical composition according to the invention is contained in the capsule in form of a loose capsule filling, i.e. not in compacted form.

The dose of Nilotinib that is contained in the pharmaceutical dosage form is not particularly limited. In preferred embodiments, the pharmaceutical dosage form contains a dose of 50 mg, 100 mg, 150 mg, 200 mg, or 250 mg, in each case expressed as equivalent weight based upon the non-salt non-solvate form of Nilotinib.

The recommended dose is (i) 300 mg twice daily in newly diagnosed patients with chronic myelogenous leukemia (CML) in the chronic phase, or (ii) 400 mg twice daily in patients with chronic or accelerated phase chronic myelogenous leukemia (CML) with resistance or intolerance to prior therapy.

The pharmaceutical dosage form according to the invention preferably provides immediate release of Nilotinib. Preferably, when determining *in vitro* dissolution in accordance with Ph. Eur. and USP, basket method, 100 rpm, 1000 mL 0.1N HCl, 37°C, after 30 minutes at least 70 wt.-%, preferably at least 80 wt.-% of the Nilotinib that was originally contained in the dosage form have been released.

Another aspect of the invention relates to the pharmaceutical dosage form according to the invention as described above for use in the treatment of cancer. The invention accordingly also relates to the use of Nilotinib of a physiologically acceptable salt and or solvate thereof for the manufacture of a pharmaceutical dosage form according to the invention as described above for treating cancer. The invention accordingly also relates to a method for treating cancer comprising administering to a subject in need thereof the pharmaceutical dosage form according to the invention as described above.

Preferably, the cancer is chronic myeloid leukemia or a gastrointestinal stromal tumor.

Preferably, the patients to be treated are adult and pediatric patients with newly diagnosed Philadelphia chromosome positive chronic myelogenous leukemia (CML) in the chronic phase; adult patients with chronic phase and accelerated phase Philadelphia chromosome positive CML with resistance or intolerance to prior therapy including Imatinib; or pediatric patients with chronic phase Philadelphia chromosome positive CML with resistance or intolerance to prior therapy including Imatinib.

Preferably, administration proceeds orally; preferably once daily or twice daily; more preferably twice daily.

Peak concentrations of Nilotinib are preferably reached 3 hours after oral administration of the pharmaceutical dosage form according to the invention. In preferred embodiments, the dosage form for use according to the invention upon oral administration provides an Coefficient of Variation (inter-patient variability) of cₘₐₓ of not more than 50%, preferably not more than 45%; and/or of AUC_{0-∞} of not more than 55%, preferably not more than 50%. Methods for determining cₘₐₓ, AUC and the Coefficient of Variation thereof are known to the skilled person and according to the invention are determined in accordance with guidance of regulatory authorities (see e.g. Guidance for Industry, Q2B Validation of Analytical Procedures: Methodology). Preferably, these parameters are determined in accordance with X. Tian et al., Clinical Pharmacokinetic and Pharmacodynamic Overview of Nilotinib, a Selective Tyrosine Kinase Inhibitor, The Journal of Clinical Pharmacology, 2018, 58(12) 1533-1540.

The following examples further illustrate the invention but are not to be construed as limiting its scope.

### Examples 1-3 (inventive):

Three pharmaceutical dosage forms having a dose strength of Nilotinib of 50 mg, 150 mg and 200 mg were prepared from different amounts of the same pharmaceutical composition. The amounts are compiled in the table here below:

| Composition | Function | Quantity per capsule | | | |
|---|---|---|---|---|---|
| | | | 1 | 2 | 3 |
| | | [%] | [mg] | [mg] | [mg] |
| Nilotinib hydrochloride (corresponding to Nilotinib base) | Drug | 53.45 | 53.45 (50) | 160.35 (150) | 213.80 (200) |
| Lactose, anhydrous | Diluent | 39.37 | 39.37 | 118.12 | 157.49 |
| Crospovidone (Type A) | Disintegrant | 3.98 | 3.98 | 11.93 | 15.91 |
| Silica, colloidal anhydrous | Glidant | 1.00 | 1.00 | 3.00 | 4.00 |
| Magnesium stearate | Lubricant | 1.50 | 1.50 | 4.50 | 6.00 |
| Poloxamer P188 | Surfactant | 0.70 | 0.70 | 2.10 | 2.80 |
| total | | 100.00 | 100.00 | 300.00 | 400.00 |

Anhydrous colloidal silica (glidant) and magnesium stearate (lubricant) were each divided into a first fraction and into a second fraction (remainder), 50:50 w/w.

The pharmaceutical composition consisted of an intragranular phase prepared by dry granulation (roller compaction) and an extragranular phase, as compiled in the following table:

| | | |
|---|---|---|
| intragranular | Nilotinib hydrochloride (corresponding to Nilotinib base) | Drug |
| | Lactose, anhydrous | Diluent |
| | Crospovidone (Type A) | Disintegrant |
| | Silica, colloidal anhydrous Part 1 | Glidant |
| | Magnesium stearate Part 1 | Lubricant |
| extragranular | Poloxamer P188 | Surfactant |
| | Silica, colloidal anhydrous Part 2 | Glidant |
| | Magnesium stearate Part 2 | Lubricant |

Anhydrous lactose, crospovidone, the first fraction of anhydrous colloidal silica and the first fraction of magnesium stearate were weighed and sifted over a sieve having a net size of 0.7 mm. Crystalline anhydrous Nilotinib hydrochloride (form T17) was added and the mixture was blended in a rotational blender (Böhle PM 400) for 10 minutes at 6 rpm.

The blend was roller compacted (Alexanderwerk WP120N) at a hydraulic pressure of about 30 bar and an average roller gap of about 1 mm. The dry granules were sieved, first net sieve 1.60 mm, second net sieve 0.80 mm.

The thus obtained calibrated granules were divided into five fractions (geometric dilution process). Poloxamer 188 was mixed with a first fraction of the calibrated granules, sieved through a 0.7 mm sieve and blended for 1 minute. A second fraction of the calibrated granules was added, sieved through a 0.7 mm sieve and blended for 1 minute. A third fraction of the calibrated granules was added, sieved through a 0.7 mm sieve and blended for 1 minute. A fourth fraction of the calibrated granules was added, sieved through a 0.7 mm sieve and blended for 1 minute. A fifth and final fraction of the calibrated granules was added, sieved through a 0.7 mm sieve and blended for 1 minute.

The thus obtained product was mixed with the second fraction (remainder) of anhydrous colloidal silica (glidant) and the mixture was blended in a rotational blender (Böhle PM 400) for 5 minutes at 6 rpm.

The thus obtained pre-blend was mixed with the second fraction (remainder) of magnesium stearate (lubricant) and the mixture was blended in a rotational blender (Böhle PM 400) for 3 minutes at 6 rpm.

The thus obtained pharmaceutical composition was filled into hard gelatin capsules of different size at the desired dose strength.

### Examples 4 and 5 (less preferred) and Examples 6 and 7 (preferred):

Four pharmaceutical compositions were prepared under the same conditions from the same starting materials in the same individual amount (content of poloxamer 0.7 wt.-%, see Example 1).

The only difference was that two different commercial poloxamers having different particle size (non-micronized [Kolliphor^{®} P 188] vs. micronized [Kolliphor^{®} P 188 micro]) were used and that the poloxamer was blended with the granules (that had been prepared by dry granulation under identical conditions) under different blending conditions (direct addition vs. geometric dilution):

| | less preferred | | preferred | |
|---|---|---|---|---|
| | Example 4 | Example 5 | Example 6 | Example 7 |
| grade poloxamer | non-micronized | micronized | non-micronized | micronized |
| blending process | direct addition | direct addition | geometric dilution | geometric dilution |

The poloxamer in the drug product was quantified by HPLC-RI. The method was developed in accordance with Y. Mao et al., Quantitation of poloxamers in pharmaceutical formulations using size exclusion chromatography and colorimetric method, Journal of Pharmaceutical and Biomedical Analysis, 35 (2004) 1127-1142. Homogeneity of distribution was quantified based upon relative standard deviation (RSD) of samples and the results are compiled in the table here below:

| | less preferred | | preferred | |
|---|---|---|---|---|
| | Example 4 | Example 5 | Example 6 | Example 7 |
| grade poloxamer | non-micronized | micronized | non-micronized | micronized |
| blending process | direct addition | direct addition | geometric dilution | geometric dilution |
| RSD | 12.6% | 11.1% | 8.6% | n.d. |

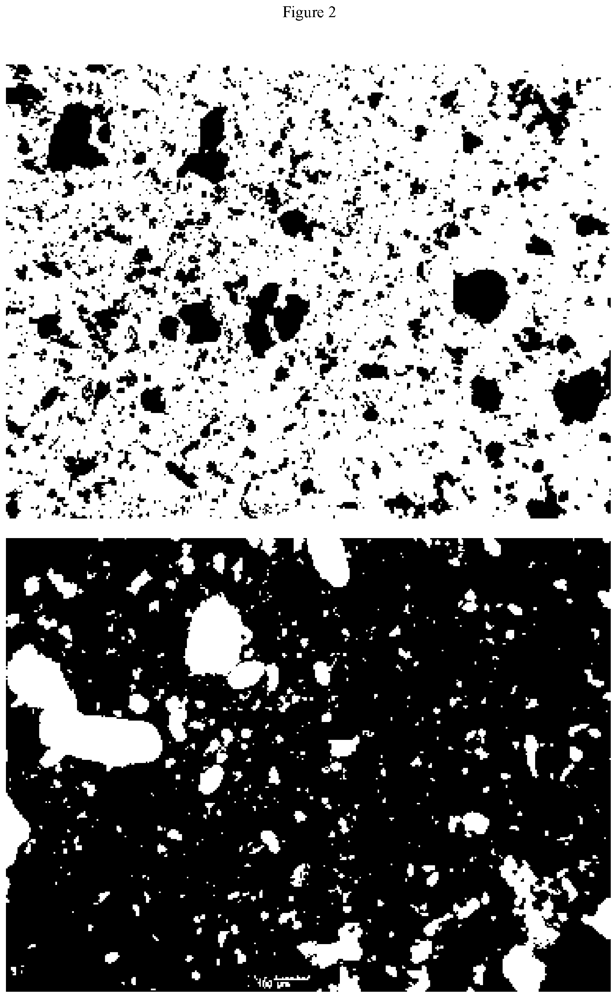

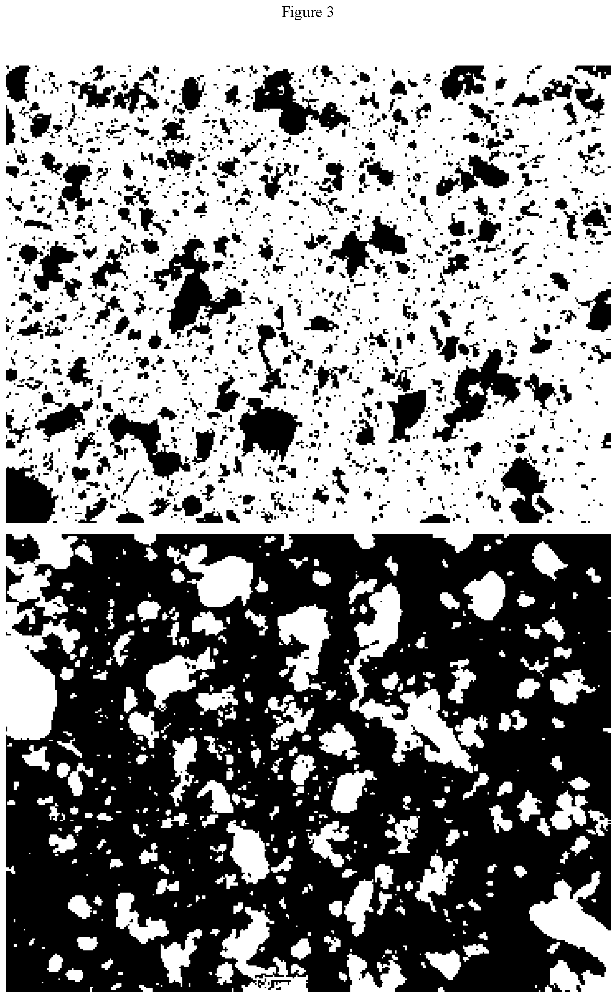

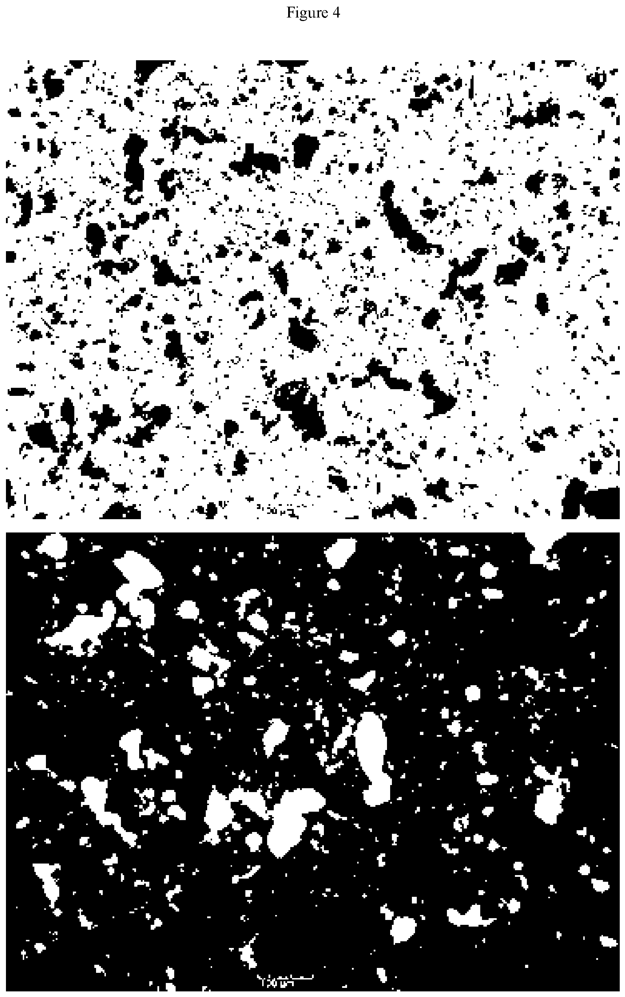

Figures 1 to 4 show photographs (10X magnitude) of pharmaceutical formulations of Examples 4-7. Figure 1 shows comparative pharmaceutical formulation of Example 4 where the granules were blended with non-micronized poloxamer by direct addition. Figure 2 shows comparative pharmaceutical formulation of Example 5 where the granules were blended with micronized poloxamer by direct addition. Figure 3 shows inventive pharmaceutical formulation of Example 6 where the granules were blended with non-micronized poloxamer by geometric dilution. Figure 4 shows inventive pharmaceutical formulation of Example 7 where the granules were blended with micronized poloxamer by geometric dilution.

As demonstrated by the above comparative data, blending the dry granules with poloxamer by geometric dilution has significant advantages that can be achieved with micronized poloxamer (Example 7) but also with non-micronized poloxamer (Example 6).

Pharmaceutical dosage forms made from the pharmaceutical compositions of Examples 4 and 7 as well as a comparative dosage form made by wet granulation where poloxamer is contained in the intragranular phase were tested in a clinical trial under fasting conditions. Coefficients of Variation (CV) of cₘₐₓ and of AUC_{0-∞} were determined and the *in vivo* data are compiled in the table here below:

| | extragranular surfactant (dry granulation) | | intragranular surfactant (wet granulation) |
|---|---|---|---|
| | Example 4 | Example 7 | comparative |
| | less preferred | preferred | |
| CV %, cₘₐₓ | 52.8 | 40.1 | 36.4 |
| CV % AUC_{0-∞} | 58.1 | 46.6 | 44.7 |

As demonstrated by the above comparative data, based upon geometric dilution principle, pharmaceutical compositions can be prepared by an anhydrous granulation process (i.e. dry granulation) using surfactant such as poloxamer in the extragranular phase and nonetheless ensuring satisfactory and decent *in vivo* data with acceptable inter subject variability.

## Claims

1. A pharmaceutical composition comprising
(i) granules comprising Nilotinib or a physiologically acceptable salt and/or solvate thereof; and
(ii) an extragranular phase;
wherein the pharmaceutical composition comprises a surfactant, wherein the surfactant is a poloxamer and wherein essentially the total amount of surfactant that is contained in the composition is contained in the extragranular phase; and
wherein the granules are dry granulated.

2. The pharmaceutical composition according to claim 1, wherein the surfactant is poloxamer 188.

3. The pharmaceutical composition according to any of the preceding claims, wherein the content of the surfactant is within the range of from 0.5 to 1.5 wt.-%, preferably 0.70±0.20 wt.-%, more preferably 0.80±0.20 wt.-%, still more preferably 0.90±0.20 wt.-%, yet more preferably 1.00±0.20 wt.-%, relative to the total weight of the composition.

4. The pharmaceutical composition according to any of the preceding claims, wherein the Nilotinib is crystalline Nilotinib hydrochloride monohydrate salt; preferably Nilotinib HCl form B.

5. The pharmaceutical composition according to any of the preceding claims, wherein the granules comprise a diluent; preferably selected from the group consisting of lactose, glucose, sucrose, sorbitol, mannitol, dextrates, dextrin, dextrose, microcrystalline cellulose, and powdered cellulose; preferably lactose.

6. The pharmaceutical composition according to claim 5, wherein the content of the diluent is within the range of 40±35 wt.-%, preferably 40±30 wt.-%, more preferably 40±25 wt.-%, still more preferably 40±20 wt.-%, yet more preferably 40±15 wt.-%, even more preferably 40±10 wt.-%, most preferably 40±5.0 wt.-%, and in particular 40±2.5 wt.-%, relative to the total weight of the composition.

7. The pharmaceutical composition according to claim 5 or 6, wherein essentially the total amount of diluent that is contained in the composition is contained in the granules.

8. The pharmaceutical composition according to any of the preceding claims, wherein the granules comprise a disintegrant; preferably selected from the group consisting of cross-linked polyvinyl pyrrolidone, cross-linked sodium carboxymethylcellulose, cross-linked calcium carboxymethylcellulose, pregelatinized starch, clay, cellulose, soy polysaccharides, alginates, and gums; preferably cross-linked polyvinyl pyrrolidone.

9. The pharmaceutical composition according to claim 8, wherein the content of the disintegrant is within the range of 4.0±3.5 wt.-%, preferably 4.0±3.0 wt.-%, more preferably 4.0±2.5 wt.-%, still more preferably 4.0±2.0 wt.-%, yet more preferably 4.0±1.6 wt.-%, even more preferably 4.0±1.4 wt.-%, most preferably 4.0±1.2 wt.-%, and in particular 4.0±1.0 wt.-%, relative to the total weight of the composition.

10. The pharmaceutical composition according to claim 8 or 9, wherein essentially the total amount of disintegrant that is contained in the composition is contained in the granules.

11. The pharmaceutical composition according to any of the preceding claims, which comprises no povidone; preferably no binder selected from the group consisting of povidone, microcrystalline cellulose, hydroxyethylcellulose, hydroxypropylcellulose, and hydroxypropylmethylcellulose.

12. A process for the preparation of a pharmaceutical composition according to any of the preceding claims, which comprises the steps of
(a) providing a formulation comprising
- Nilotinib or a physiologically acceptable salt and/or solvate thereof, preferably crystalline Nilotinib or a physiologically acceptable salt and/or solvate thereof, more preferably crystalline anhydrous Nilotinib hydrochloride or crystalline Nilotinib hydrochloride monohydrate,
- optionally diluent,
- optionally disintegrant,
- optionally a fraction of glidant, and
- optionally a fraction of lubricant;
(b) dry granulating the formulation thereby obtaining granules;
(c) optionally, sieving the granules;
(d) providing a surfactant formulation comprising or essentially consisting of poloxamer; and
(e) blending the surfactant formulation and the granules.

13. The process according to claim 12, wherein in step (e) in an initial sub-step the surfactant formulation is blended with a fraction of the granules, and wherein in subsequent sub-steps additional fractions of the granules are added to the blend.

14. A pharmaceutical dosage form comprising a pharmaceutical composition according to any of claims 1 to 11.

## Patentansprüche

1. Pharmazeutische Zusammensetzung, umfassend
(i) Granulate, die Nilotinib oder ein physiologisch verträgliches Salz und/oder Solvat davon umfassen; und
(ii) eine extragranulare Phase
wobei die pharmazeutische Zusammensetzung ein Tensid umfasst, wobei das Tensid ein Poloxamer ist und wobei im Wesentlichen die Gesamtmenge an Tensid, die in der Zusammensetzung enthalten ist, in der extragranularen Phase enthalten ist; und
wobei die Granulate trockengranuliert sind.

2. Pharmazeutische Zusammensetzung nach Anspruch 1, wobei das Tensid Poloxamer 188 ist.

3. Die pharmazeutische Zusammensetzung gemäß einem der vorstehenden Ansprüche, wobei der Gehalt des Tensids im Bereich von 0,5 bis 1,5 Gew.-%, vorzugsweise 0,70 ± 0,20 Gew.-%, noch bevorzugter 0,80 ± 0,20 Gew.-%, noch bevorzugter 0,90 ± 0,20 Gew.-%, noch bevorzugter 1,00 ± 0,20 Gew.-%, bezogen auf das Gesamtgewicht der Zusammensetzung.

4. Die pharmazeutische Zusammensetzung gemäß einem der vorstehenden Ansprüche, wobei das Nilotinib kristallines Nilotinibhydrochlorid-Monohydratsalz ist, vorzugsweise Nilotinib-HCl-Form B.

5. Die pharmazeutische Zusammensetzung gemäß einem der vorstehenden Ansprüche, wobei das Granulat ein Verdünnungsmittel umfasst, vorzugsweise ausgewählt aus der Gruppe bestehend aus Lactose, Glucose, Saccharose, Sorbit, Mannit, Dextraten, Dextrin, Dextrose, mikrokristalliner Cellulose und pulverförmiger Cellulose, vorzugsweise Lactose.

6. Die pharmazeutische Zusammensetzung gemäß Anspruch 5, wobei der Gehalt des Verdünnungsmittels im Bereich von 40 ± 35 Gew.-%, vorzugsweise 40 ± 30 Gew.-%, noch bevorzugter 40 ± 25 Gew.-%, noch bevorzugter 40 ± 20 Gew.-%, noch bevorzugter 40 ± 15 Gew.-%, noch bevorzugter 40 ± 10 Gew.-%, am meisten bevorzugt 40 ± 5,0 Gew.-% und insbesondere 40 ± 2,5 Gew.- % beträgt, bezogen auf das Gesamtgewicht der Zusammensetzung.

7. Die pharmazeutische Zusammensetzung gemäß Anspruch 5 oder 6, wobei im Wesentlichen die Gesamtmenge an Verdünnungsmittel, die in der Zusammensetzung enthalten ist, in den Granulaten enthalten ist.

8. Die pharmazeutische Zusammensetzung gemäß einem der vorstehenden Ansprüche, wobei das Granulat ein Sprengmittel umfasst, das vorzugsweise aus der Gruppe ausgewählt ist, die aus vernetztem Polyvinyl pyrrolidon, vernetzter Natriumcarboxymethylcellulose, vernetzter Calciumcarboxymethylcellulose, vorgelatinierter Stärke, Ton, Cellulose, Sojapolysacchariden, Alginaten und Gummi besteht, vorzugsweise vernetztem Polyvinylpyrrolidon.

9. Die pharmazeutische Zusammensetzung gemäß Anspruch 8, wobei der Gehalt des Sprengmittels im Bereich von 4,0 ± 3,5 Gew.-%, vorzugsweise 4,0 ± 3,0 Gew.-%, noch bevorzugter 4,0 ± 2,5 Gew.-%, noch bevorzugter 4,0 ± 2,0 Gew.-%, noch bevorzugter 4,0 ± 1,6 Gew.-%, noch bevorzugter 4,0 ± 1,4 Gew.-%, am meisten bevorzugt 4,0 ± 1,2 Gew.-% und insbesondere 4,0 ± 1,0 Gew.-% beträgt, bezogen auf das Gesamtgewicht der Zusammensetzung.

10. Die pharmazeutische Zusammensetzung gemäß Anspruch 8 oder 9, wobei im Wesentlichen die Gesamtmenge an Zerfallsmittel, die in der Zusammensetzung enthalten ist, in den Granulaten enthalten ist.

11. Die pharmazeutische Zusammensetzung gemäß einem der vorstehenden Ansprüche, die kein Povidon enthält; vorzugsweise kein Bindemittel, ausgewählt aus der Gruppe bestehend aus Povidon, mikrokristalliner Cellulose, Hydroxyethylcellulose, Hydroxypropylcellulose und Hydroxypropylmethylcellulose.

12. Verfahren zur Herstellung einer pharmazeutischen Zusammensetzung gemäß einem der vorstehenden Ansprüche, das die folgenden Schritte umfasst
(a) Bereitstellen einer Formulierung, die umfasst
- Nilotinib oder ein physiologisch verträgliches Salz und/oder Solvat davon, vorzugsweise kristallines Nilotinib oder ein physiologisch verträgliches Salz und/oder Solvat davon, noch bevorzugter kristallines wasserfreies Nilotinibhydrochlorid oder kristallines Nilotinibhydrochlorid-Monohydrat,
- gegebenenfalls ein Verdünnungsmittel,
- optional Sprengmittel,
- optional ein Anteil an Gleitmittel und
- optional ein Anteil an Gleitmittel;
(b) Trockengranulieren der Formulierung, wodurch Granulat erhalten wird;
(c) optional, Sieben der Granulate;
(d) Bereitstellen einer Tensidformulierung, die Poloxamer umfasst oder im Wesentlichen daraus besteht; und
(e) Vermischen der Tensidformulierung und des Granulats.

13. Verfahren nach Anspruch 12, wobei in Schritt (e) in einem ersten Teilschritt die Tensidformulierung mit einem Teil der Granulate gemischt wird und wobei in nachfolgenden Teilschritten weitere Teile der Granulate zu der Mischung hinzugefügt werden.

14. Pharmazeutische Darreichungsform, umfassend eine pharmazeutische Zusammensetzung gemäß einem der Ansprüche 1 bis 11.

## Revendications

1. Composition pharmaceutique comprenant
(i) des granulés comprenant du nilotinib ou un sel physiologiquement acceptable et/ou un solvate de celui-ci ; et
(ii) une phase extragranulaire ;
dans laquelle la composition pharmaceutique comprend un tensioactif, dans laquelle le tensioactif est un poloxamère et dans laquelle essentiellement la quantité totale du tensioactif qui est contenu dans la composition est contenu dans la phase extragranulaire ; et
dans laquelle les granulés sont granulés par voie sèche.

2. Composition pharmaceutique selon la revendication 1, dans laquelle le tensioactif est le poloxamère 188.

3. Composition pharmaceutique selon l'une quelconque des revendications précédentes, dans laquelle la teneur en tensioactif est située dans la plage allant de 0,5 à 1,5 % en poids, de préférence de 0,70 ± 0,20 % en poids, de préférence encore de 0,80 ± 0,20 % en poids, encore plus préférentiellement de 0,90 ± 0,20 % en poids, et encore plus préférentiellement de 1,00 ± 0,20 % en poids, par rapport au poids total de la composition.

4. Composition pharmaceutique selon l'une quelconque des revendications précédentes, dans laquelle le nilotinib est le sel de chlorhydrate monohydraté de nilotinib cristallin ; de préférence la forme B du HCl de nilotinib.

5. Composition pharmaceutique selon l'une quelconque des revendications précédentes, dans laquelle les granulés comprennent un diluant ; choisi de préférence dans le groupe constitué par du lactose, du glucose, du saccharose, du sorbitol, du mannitol, des dextrates, de la dextrine, du dextrose, de la cellulose microcristalline et de la cellulose en poudre ; de préférence du lactose.

6. Composition pharmaceutique selon la revendication 5, dans laquelle la teneur en diluant est située dans la plage de 40 ± 35 % en poids, de préférence de 40 ± 30 % en poids, de préférence encore de 40 ± 25 % en poids, encore plus préférentiellement de 40 ± 20 % en poids, encore plus préférentiellement de 40 ± 15 % en poids, encore plus préférentiellement de 40 ± 10 % en poids, de manière préférée entre toutes de 40 ± 5,0 % en poids, et en particulier de 40 ± 2,5 % en poids, par rapport au poids total de la composition.

7. Composition pharmaceutique selon la revendication 5 ou 6, dans laquelle essentiellement la quantité totale du diluant qui est contenu dans la composition est contenu dans les granulés.

8. Composition pharmaceutique selon l'une quelconque des revendications précédentes, dans laquelle les granulés comprennent un délitant ; de préférence choisi dans le groupe constitué par de la polyvinylpyrrolidone réticulée, de la carboxyméthylcellulose sodique réticulée, de la carboxyméthylcellulose calcique réticulée, de l'amidon prégélatinisé, de l'argile, de la cellulose, des polysaccharides de soja, des alginates et des gommes ; de préférence de la polyvinylpyrrolidone réticulée.

9. Composition pharmaceutique selon la revendication 8, dans laquelle la teneur en délitant est située dans la plage de 4,0 ± 3,5 % en poids, de préférence de 4,0 ± 3,0 % en poids, de préférence encore de 4,0 ± 2,5 % en poids, encore plus préférentiellement de 4,0 ± 2,0 % en poids, encore plus préférentiellement de 4,0 ± 1,6 % en poids, encore plus préférentiellement de 4,0 ± 1,4 % en poids, de manière préférée entre toutes de 4,0 ± 1,2 % en poids, et en particulier de 4,0 ± 1,0 % en poids, par rapport au poids total de la composition.

10. Composition pharmaceutique selon la revendication 8 ou 9, dans laquelle essentiellement la quantité totale du délitant qui est contenu dans la composition est contenu dans les granulés.

11. Composition pharmaceutique selon l'une quelconque des revendications précédentes, qui ne comprend pas de povidone ; qui ne comprend de préférence pas de liant choisi dans le groupe consisté par la povidone, la cellulose microcristalline, l'hy-droxyéthylcellulose, l'hydroxypropylcellulose et l'hydroxypro-pylméthylcellulose.

12. Procédé de préparation d'une composition pharmaceutique selon l'une quelconque des revendications précédentes, qui comprend les étapes consistant à
(a) fournir une formulation comprenant
- du nilotinib ou un sel physiologiquement acceptable et/ou un solvate de celui-ci, de préférence du nilotinib cristallin ou un sel physiologiquement acceptable et/ou un solvate de celui-ci, de préférence encore du chlorhydrate de nilotinib anhydre cristallin ou du chlorhydrate de nilotinib monohydraté cristallin,
- éventuellement un diluant,
- éventuellement un délitant,
- éventuellement une fraction de glissant, et
- éventuellement une fraction de lubrifiant ;
(b) granuler la formulation par voie sèche afin d'obtenir ainsi des granulés ;
(c) éventuellement, tamiser les granulés ;
(d) fournir une formulation de tensioactif comprenant ou constituée essentiellement de poloxamère ; et
(e) mélanger la formulation de tensioactif et les granulés.

13. Procédé selon la revendication 12, dans lequel, à l'étape (e), dans une sous-étape initiale, la formulation de tensioactif est mélangée avec une fraction des granulés, et dans lequel, dans les sous-étapes suivantes, des fractions supplémentaires des granulés sont ajoutées au mélange.

14. Forme médicamenteuse pharmaceutique comprenant une composition pharmaceutique selon l'une quelconque des revendications 1 à 11.
